# EUROPEAN PATENT SPECIFICATION

(11) **EP 3 297 424 B1**
(45) Date of publication and mention of the grant of the patent: **07.09.2022**
(21) Application number: 16796022.8
(22) Date of filing: 19.05.2016
(51) Int. Cl.: A01G 25/16, G01J 5/00, G01J 5/10, G01N 33/00

(54) **METHOD AND SYSTEM FOR EVALUATING CROP IRRIGATION CONDITION USING THERMAL IMAGING**
VERFAHREN UND SYSTEM ZUR BEURTEILUNG DES PFLANZENBEWÄSSERUNGSZUSTANDS MITTELS THERMISCHER BILDGEBUNG
PROCÉDÉ ET SYSTÈME D'ÉVALUATION DE CONDITION D'IRRIGATION DE CULTURES À L'AIDE DE L'IMAGERIE THERMIQUE

(30) Priority: 19.05.2015 US 201562163475 P
(43) Date of publication of application: 28.03.2018
(73) Proprietor: Saturas Ltd, 2017400 Misgav Industrial Park (IL)
(72) Inventor: MERON, Moshe, Upper Galillee 1213500 (IL)
(74) Representative: Brevalex
(86) International application number: PCT/IL2016/050530
(87) International publication number: WO 2016/185477

(56) References cited:
- WO-A1-98/04915
- WO-A1-98/04915
- WO-A2-2010/121176
- WO-A2-2010/121176
- US-A1- 2014 326 801
- US-A1- 2014 326 801
- The Trendlines Group: "Stem water potential (SWP) sensor for optimal irrigation -- Saturas", Youtube, 15 February 2015 (2015-02-15), page 1, XP054977027, Retrieved from the Internet: URL:https://www.youtube.com/watch?v=BQRFw8 IXafk [retrieved on 2016-12-21]
- SATURAS: 'Stem water potential (SWP) sensor for optimal irrigation' YOUTUBE ONLINE VIDEO, [Online] 15 February 2015, XP054977027 Retrieved from the Internet: <URL:https://www.youtube.com/watch?v=BQRFw8 1Xafk>

## Description

### FIELD OF THE INVENTION

The present invention generally relates to a system and method for evaluating irrigation condition in crops using thermal imagery.

### BACKGROUND OF THE INVENTION

Various methods and systems are currently used in the agricultural industry for measuring various parameters indicative of water stress in crops for improving crop irrigation.

Some of these systems include infrared (IR) based thermal mapping for measuring water stress in the plants of the crop. This mapping requires placing one or more thermal imaging devices such as IR cameras in the crop field(s) and acquiring thermal images of the crop. To deduce the water stress from the thermal image of the crop, a temperature index must be used to calibrate the measured data from the IR camera. This index (also called crop water stress index (CWSI) see http://www.israelagri.com/?CategoryID=396&ArticleID=645) is obtained typically by using a reference temperature measurement (e.g. obtained by using a thermometer) typically measuring temperature of the air in the crop area.

To actually measure the water potential inside the plants of the crop, at least a reasonable amount of plants in the crop must be equipped with a water potential sensor. Some of these sensors or detector require insertion thereof into the plant stem or measure water potential on the ground near the plant stem.

One method for inserting water potential sensors into a plant stem is described in Legge *et al.,* 1985. In this paper, an osmotic tensiometer is inserted into a tree by hammering a hole in the bark by steel punching thereof and filling it with water above the punch level to keep the tissue in the hole wetted. In this case, the hole was filled with caulking compound after the senor was installed in the hole formed in the tree bark.

Variable-rate irrigation by machines or solid set systems has become technically feasible, however mapping crop water status is necessary to match irrigation quantities to site-specific crop water demands (Meron *et al.,* 2010). Remote thermal sensing can provide such maps in sufficient detail and in a timely way. Digital crop water stress maps can be generated using geo-referenced high-resolution thermal imagery and artificial reference surfaces. Canopy-related pixels can be separated from those of the soil by upper and lower thresholds related to air temperature, and canopy temperatures calculated from the coldest 33% of the pixel histogram. Artificial surfaces can be wetted for providing reference temperatures for the crop water stress index (CWSI) normalization to ambient conditions. US 2014/326801 A1 discloses an apparatus and system of sensors and methods that monitor leaf temperature and corresponding microclimatic conditions in real-time and produce a reliable plant water status indicator.

### SUMMARY OF THE INVENTION

The present invention provides a system for evaluating irrigation condition in crops using thermal imagery as defined by the subject-matter of claims 1 to 5.

The present invention further provides a method for evaluating irrigation condition in crops using thermal imagery as defined by the subject-matter of claims 6 to 7.

### BRIEF DESCRIPTION OF THE DRAWINGS

Fig. 1 schematically illustrates a method for installing a water potential detector in a plant stem, using two-stage drilling, not in accordance with the present invention.
**Figs. 2A** and **2B** show two types of drill bits each used for a different stage in the installation process: **Fig. 2A** shows the first type drill bit having a central spur and two wings with recesses for rough drilling in the stem; and **Fig. 2B** shows a smoothing second type drill bit having wings with smooth surface and no spur or recesses.
**Fig. 3** shows a system for evaluating irrigation condition in crops using thermal imagery, according to the invention.
**Fig. 4** is a flowchart showing a method for evaluating irrigation condition (water stress) in crops using thermal imagery by using one or more water potential detectors for calibrating the thermal system, according to the invention.

### DETAILED DESCRIPTION OF SOME EMBODIMENTS OF THE INVENTION

In the following detailed description of various embodiments, reference is made to the accompanying drawings that form a part thereof, and in which are shown by way of illustration specific embodiments in which the invention may be practiced. It is understood that other embodiments may be utilized and structural changes may be made without departing from the scope of the present invention.

The present description describes methods and systems, not in accordance with the invention, for installing a water potential detector in a plant stem using a unique two-stage drilling technique for forming a smooth bore in the plant stem without damaging the stem tissue. The water potential detector is configured for measuring stem water potential via direct fluid-to-fluid contact and therefore requires the bore to be adjacent to the stem vascular conduit. To allow such direct fluid-to-fluid contact the detector has a compartment with an osmoticum such as PolyEthyleneGlycol (PEG) therein, at least one selective barrier for selective transfer of fluids between the plant tissue and the osmoticum such as a membrane, and a pressure sensor configured for detecting changes in pressure of fluid in said compartment, where the water potential detector is configured for measuring water potential through direct contact between the osmoticum in the compartment and the plant tissue adjacent to the vascular conduit of the plant stem via said at least one selective barrier.

It should be noted that the skilled artisan would clearly understand that when reference is made to "at least one selective barrier for selective transfer of fluids between the plant tissue and the osmoticum", at least two, three, four or more selective barrier layers can be used. Said selective barrier layers can either be the same or different, each having different properties and purposes. For instance, two selective barrier layers can be used, one is a membrane and the other is a rigid porous structure externally covering said membrane.

According to some embodiments, the installation method includes maintaining the one or more selective barriers of the water potential detector wet throughout the delivery of the water potential detector to the plant site and throughout its installation in the plant stem; forming a bore through the plant stem by drilling therein, using a drill bit of a first type; smoothening the inner walls of the bore by using a drill bit of a second type; inserting the water potential detector into the smoothened bore such that its one or more selective barriers is in direct contact with the stem tissue of the plant; and filling the gap between the water potential detector and the stem tissue with a fluid conducting material such as fluid conducting gel or caulking materials. The plant tissue is also kept wet throughout the installation process until the bore is filled.

The method is preferably, yet not necessarily, intended for plants having thick and solid stems such as for trees and vines allowing drilling through their stem tissue using one or more drilling tools such as drillers without damaging their vascular conduit tissue.

In the first stage of the drilling, the stem is drilled to a depth therein that is proximate or adjacent to the vascular conduit tissue (e.g. until reaching the xylem apoplast). According to some embodiments, a first type of drill bit is used to perform this first stage drilling, has a central spur and a spiral bit body or wings having one or more recesses thereover for advancing the bit into the stem and for forming the bore in optimal precision to avoid damaging the tissue to an extent that will harm the plant.

According to some embodiments, a second type of drill bit is used to perform the smoothing second drilling stage for improving the direct contact between the selective barrier of the detector and the plant tissue for the direct fluid-to-fluid osmosis between the stem water and the osmoticum in the detector's compartment. This second drill bit may be designed as a spiral bit of bit having wings with smooth side surfaces having no spurs or recesses.

In yet another embodiment, before drilling begins the bark is removed, e.g. with a hole-punch, thereby allowing either easier drilling with the first type of bit, or eliminating completely the need of such a first type of bit. Alternatively, the entire drilling step may be omitted and only a bark-removal step is performed, i.e. when it is sufficient to expose the plant inner tissue, e.g. as in plants with thin stems.

Once the bore is completed, the detector is placed therein while keeping the bore (inner stem tissue) and the detector's selective barrier(s) wet. Once positioned inside the bore the bore is filled with fluid conducting filling material and the detector is fastened to the stem or attached thereto using any fastening or attachment means known in the art such as screws, glues and the like.

In certain embodiments, the bore is filled with plant hormone(s) and/or growth substances, to assist and accelerate callus formation and callus growth at the sensor-tissue interface. This enables faster healing of the plant after insertion of the sensor/device, reduce potential damage to the plant, e.g. due to pathogens or pests, and aids in preventing rejection and removal of the sensor/device from the stem.

Plant hormone(s) and/or growth substances which can be used in accordance with the present method include, but are not limited to, abscisic acid; auxins; cytokinins; ethylene; gibberellins; brassinosteroids; salicylic acid; jasmonates; plant peptide hormones; polyamines; nitric oxide (NO); strigolactones; and karrikins.

Accordingly, in certain embodiments not in accordance with the present invention, the installation method comprises the steps of: (a) providing a water potential detector as described above configured for measuring water potential through direct contact with plant tissue adjacent to the vascular conduit of the plant stem; (b) maintaining said at least one selective barrier of said water potential detector wet throughout the delivery thereof to the plant site and throughout its installation in the plant stem; (c) removing the plant's bark; (d) forming a bore through the plant stem by drilling therein, using a first type of drill bit; (e) smoothening the inner walls of the bore by using a second type of drill bit; (f) inserting the water potential detector into the smoothened bore such that said at least one selective barrier thereof is in direct contact with the stem tissue of the plant; (g) maintaining the stem tissue in the bore wet throughout the installation process; and (f) filling the gap between the water potential detector and the stem tissue with a fluid conducting material as well as plant hormone(s) and/or growth substances, wherein said plant hormone(s) and/or growth substances may be inserted into said smoothened bore before said potential detector is inserted therein.

Once installed in the plant stem, the detector can be activated and optionally connected via wires or wirelessly to a control device that is capable of reading the output data from the sensor thereof.

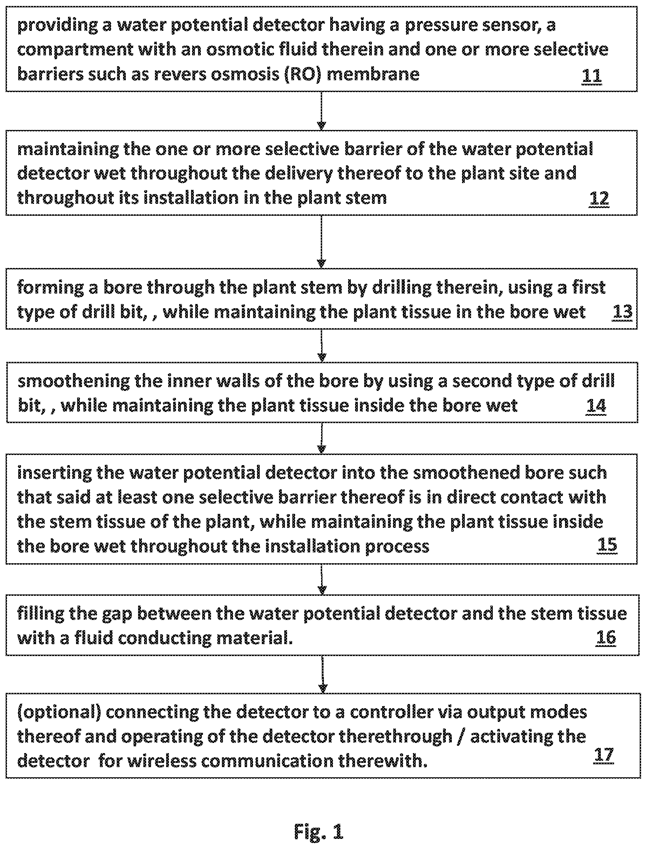

Reference is now made to Fig. 1, schematically illustrating a method for installing a water potential detector in a plant stem such as in a tree trunk, according to some embodiments not in accordance with the invention. In the process, a water potential detector is provided and used 11 having a compartment with an osmoticum therein, one or more selective barriers for selective transfer of fluids between the plant tissue and the osmoticum and a pressure sensor configured for sensing changes in pressure of fluid in the compartment, the water potential detector being configured for measuring water potential through direct contact with plant tissue adjacent to the vascular conduit of the plant stem via the one or more selective barriers.

In some embodiments, the selective barrier(s) used is a membrane such as a reverse osmosis (RO) membrane, forward osmosis (FO) membrane or a Nano filtration (NF) membrane.

Other additional selective barriers may be used for filtering larger particles in the stem fluid from penetrating into the compartment such as a rigid porous structure externally covering the membrane.

The osmoticum used in the detector may be for example water absorbent hydrogel such as PolyEthyleneGlycol (PEG).

In some embodiments, the pressure sensor of the water potential detector used can be for instance, a piezoelectric transducer sensor, a strain gauge sensor or a combination thereof.

To install the water potential detector in the plant stem, the detector is transferred to the plant site while maintaining the at least one selective barrier thereof wet throughout the delivery thereof to the plant site and throughout its installation in the plant stem **12.** To maintain the selective barrier(s) wet it may be either kept in a container having fluid (e.g. water) therein or be injected with water during the delivery thereof. For example one person may hold the detector through its delivery and another may inject water thereover for maintaining its membrane wet. An injector (e.g. syringe) with water may be also used during the drilling and placement process.

A bore in the plant stem is formed in the stem (e.g. in the tree trunk) **13** by drilling therein, using a first type of drill bit, which has a central spur and is configured for rough drilling to form the initial bore. This bore is formed such that its deepest edge is adjacent to the vascular conduit (e.g. xylem) of the stem for allowing the fluid transfer therefrom. The inner walls of the initial bore that was formed are then smoothened by using a second type of drill bit having for example smoothened edged wings **14.** The drilling is done while constantly keeping the inner plant stem tissue wetted e.g. by using a syringe or a water hose for wetting the bore that is formed while drilling thereof.

Once the bore is formed and completed the water potential detector is inserted into therein such that its one or more selective barriers are in direct contact with the stem tissue of the plant **15.** Once the detector is in place inside the completed smoothed bore, the gap between the water potential detector and the stem tissue is filled using a fluid conducting material **16** such as fluid conducting gel or caulking material.

Optionally, the detector is attached to the plant stem using fastening or attachment means such as screws, gluing materials and the like **17.**

According to some embodiments, the water potential detector used has a micro electro-mechanical system (MEMS) device embedded therein which includes the pressure sensors, a data processor and a communication unit including a transmitter and optionally also a receiver for communicating with a remote control device or system for transmitting thereto the measured potential data. The MEMS of the detector mat require electronically connecting to output nodes thereof for communicating therewith.

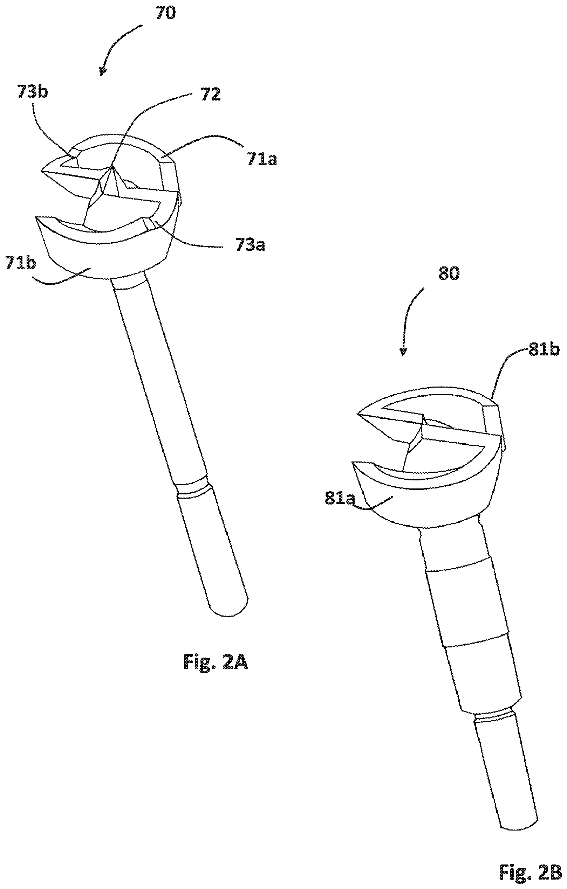

Figs. 2A and 2B show two types of drill bits **70** and **80** that can be used for the first and second stages of drilling, respectively: Fig. 2A shows the first type drill bit **70** having a central spur **72** and two wings **71a** and **71b** with recesses **73a** and **73b** thereover for rough drilling in the stem; and Fig. 2B shows a smoothing second type drill bit **80** having wings **81a** and **81b** with smooth sided surfaces and no spur or recesses thereover for smoothening the bore walls formed by using the first drill bit **70.**

Other types of drill bits can be used such as spiral bits one having a central spur and recesses thereover and the other with smoothened spiral head and no central spur.

The present invention provides systems and methods for evaluating irrigation condition (e.g. water stress) in plant crops using one or more thermal imagery systems configured for thermal mapping of an area by also using one or more water potential detectors configured for measuring water potential in a plant stem for calibration of the data from the one or more thermal imagery systems. To evaluate the overall irrigation condition of the crop at each given timeframe a central unit is used. The central unit is configured for receiving thermal imaging data indicative of acquired crop temperature maps from the thermal imagery system(s), receiving data from the at least one water potential detector and for processing the received data for evaluating irrigation condition of the crop using the data from the at least one water potential detector reference at least for calibrating the data from the thermal imagery system.

According to some embodiments, the water potential detector is configured for detecting stem water potential via direct fluid-to-fluid contact between an osmoticum therein and the stem water (e.g. water of the vascular conduit of the plant). The detector can be installed inside the plant stem of one of the plants in the crop or positioned on the ground in the crop felid.

Optionally, the water potential detector is equipped with a thermometer for direct temperature measurement.

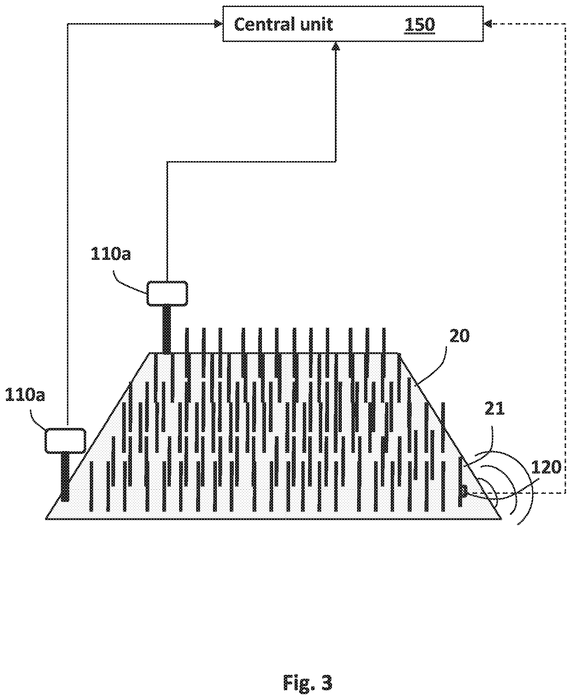

Reference is now made to Fig. 3 showing a system for evaluating water stress in a crop 20, according to some embodiments of the invention. The system includes several thermal imagery systems **110a** and **110b** for covering the entire crop field and a water potential detector **120** installed in a plant stem **21** of the crop and configured for wirelessly transmitting signals indicative of its water potential and optionally also of its thermal measurements. Each of the thermal imagery systems **110a** or **110b** is positioned and configured for thermal mapping of an area of the crop field and transmitting data indicative thereof to a central unit **150.** The central unit **150** is configured for receiving data from the one or more water potential detectors in the field such as from detector **120** and data from the one or more thermal imagery systems in the field such as systems **110a** and **110b** and processing the received data to calculate the water stress in the crop or in areas thereof.

The data from the one or more water potential detectors **120** is used at least for calibrating the crop water stress index (CWSI) of the thermal imagery systems **110a** and **110b.**

Each of the thermal imagery systems **110a** and **110b** includes a thermal camera.

The data from the one or more water potential detectors **120,** also referred to herein as "the reference data" provides the needed absolute ground stress or temperature reference for the calibration of the output of the thermal imagery systems. All other temperature levels can be related to pixel values of the imagery systems outputs even when using non-radiometric and therefore much less expensive thermal cameras for the imagery systems.

The water potential detector used for the calibration of the imagery system(s) output may be the detector described above, comprising a compartment with an osmoticum and at least one selective barrier for measuring water potential in the plant stem in which it is installed via direct fluid osmosis. The water potential detector may be configured for communicating with the central unit 150 via at least one communication link for transmitting data thereto indicative of the measured water potential. This link may be wireless communication link e.g. using radio frequency (RF) communication technologies such as WiFi, ZigBee or any other wireless communication technology known in the art.

Additionally or alternatively, the detector is configured for communication with the central unit **150** via cabled communication for transmission of the reference data.

The detector **120** may transmit measured water potential values to the central unit **150** to be used as a reference water stress value for reference data. This measure may be used for calculating the reference temperature for the CWSI.

According to some embodiments, the water potential detector **120** also comprises a thermometer and is configured for transmitting direct temperature measurements to the central unit to be used as reference data.

The water potential detector **120** may also include one or more batteries as power source. Alternatively, it may have an external solar panel, or may receive power from an external source, e.g. via the wire connection to said central unit.

In some embodiments, the central unit **150** is further configured for controlling irrigation of the crop plants according to the evaluated irrigation condition of the crop and may therefore include irrigation control means.

Additionally or alternatively, the central unit **150** is configured for transmitting data indicative of the evaluated irrigation condition of the crop and/or irrigation recommendation plan based thereon to a separate irrigation system for controlling irrigation of the crop according to the evaluated irrigation condition thereof.

In some embodiments, multiple water potential detectors can be used to cover a large field area, each detector may be installed in a different plant of the crop at locations that are adapted to optimize measurements in relation to the number of water potential detectors and the size of the crop area and crop type.

In a specific embodiment, multiple water potential detectors can be used in a single plant, preferably a large branched plant, each detector installed in a different branch/stem of the plant to optimize measurements in said plant.

The central unit **150** may include a computer having processing and communication means having a designated control application operable therethrough for carrying out the data communication and processing using at least one evaluation algorithm for the irrigation condition evaluation and calibration.

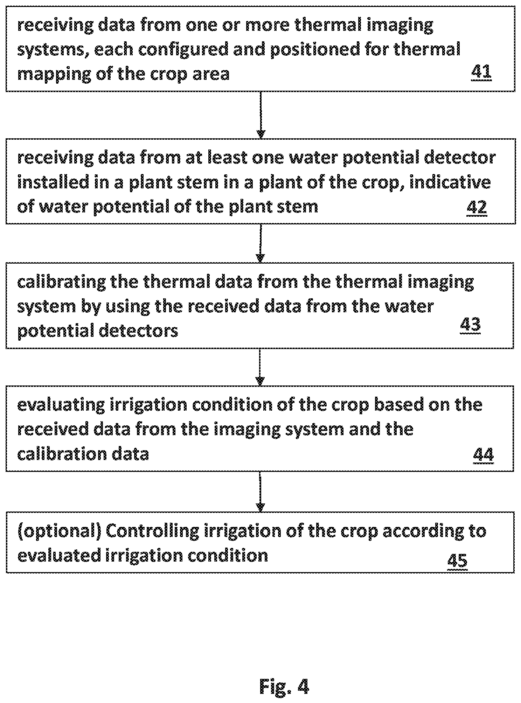

Fig. 4 is a flowchart showing a method for evaluating irrigation condition (water stress) in crops using thermal imagery by using one or more water potential detectors for calibrating the thermal system, according to other embodiments of the invention. The steps of this method can be carried out by a single processor of the central unit of the system. The data from the one or more imagery systems and from the one or more water potential detectors is received **41-42** and the thermal mapping is calibrated by using the data from the one or more water potential detectors **43.** The irrigation condition (water stress) of the crop is then evaluated based on the calibrated thermal mapping of the crop fields.

Optionally, the crop is irrigated according to the updated and calibrated thermal mapping **45** by having the central unit also control irrigation or by having the central unit transmitting the calibrated mapping data to an irrigation system controlling irrigation of the respective crop.

Any number of imagery systems and/or water potential detectors can be used depending on field size and plant type.

The detectors can be installed in the plants stems or be located on the ground for temperature and/or water potential measurements.

Many alterations and modifications may be made by those having ordinary skill in the art without departing from the spirit and scope of the invention. Therefore, it must be understood that the illustrated embodiment has been set forth only for the purposes of example and that it should not be taken as limiting the invention as defined by the following invention and its various embodiments and/or by the following claims. For example, notwithstanding the fact that the elements of a claim are set forth below in a certain combination, it must be expressly understood that the invention includes other combinations of fewer, more or different elements, which are disclosed in above even when not initially claimed in such combinations. A teaching that two elements are combined in a claimed combination is further to be understood as also allowing for a claimed combination in which the two elements are not combined with each other, but may be used alone or combined in other combinations. The excision of any disclosed element of the invention is explicitly contemplated as within the scope of the invention.

The words used in this specification to describe the invention and its various embodiments are to be understood not only in the sense of their commonly defined meanings, but to include by special definition in this specification structure, material or acts beyond the scope of the commonly defined meanings. Thus if an element can be understood in the context of this specification as including more than one meaning, then its use in a claim must be understood as being generic to all possible meanings supported by the specification and by the word itself.

The definitions of the words or elements of the following claims are, therefore, defined in this specification to include not only the combination of elements which are literally set forth, but all equivalent structure, material or acts for performing substantially the same function in substantially the same way to obtain substantially the same result. In this sense it is therefore contemplated that an equivalent substitution of two or more elements may be made for any one of the elements in the claims below or that a single element may be substituted for two or more elements in a claim. Although elements may be described above as acting in certain combinations and even initially claimed as such, it is to be expressly understood that one or more elements from a claimed combination can in some cases be excised from the combination and that the claimed combination may be directed to a sub-combination or variation of a sub-combination.

Insubstantial changes from the claimed subject matter as viewed by a person with ordinary skill in the art, now known or later devised, are expressly contemplated as being equivalently within the scope of the claims. Therefore, obvious substitutions now or later known to one with ordinary skill in the art are defined to be within the scope of the defined elements.

The claims are thus to be understood to include what is specifically illustrated and described above, what is conceptually equivalent, what can be obviously substituted and also what essentially incorporates the essential idea of the invention.

Although the invention has been described in detail, nevertheless changes and modifications, which do not depart from the teachings of the present invention, will be evident to those skilled in the art. Such changes and modifications are deemed to come within the purview of the present invention and the appended claims.

### REFERENCES

1. N. J. Legge and D. J. Connor, "Hydraulic Characteristics of Mountain Ash (Eucalyptus regnans F. Muell.) derived from in situ Measurements of Stem Water Potential", Aust. J. Plant Physiol., 1985, 12, pp. 77-88.
2. Meron M., Tsipris J., Orlov V., Alchanati V. and Cohen Y., "Crop water stress mapping for site-specific irrigation by thermal imagery and artificial reference surfaces", Precision Agriculture, April 2010, Volume 11, Issue 2, pp 148-162.

## Claims

1. A system for evaluating irrigation condition in crops using thermal imagery, said system comprising:
a) at least one thermal imagery system (110a) configured for thermal mapping of an area;
b) at least one water potential detector (120) configured for measuring water potential in a plant stem (21) in which it is installed and transmitting data indicative of its measurements; and
c) a central unit (150) configured for receiving thermal imaging data indicative of acquired crop temperature maps, receiving data from the at least one water potential detector (120) and for processing the received data for evaluating irrigation condition of the crop, **characterized in that**
the central unit (150) is configured for evaluating irrigation condition in crops using the data from the at least one water potential detector reference for calibrating the data from the thermal imagery system (110a).

2. The system according to claim 1, wherein each of said at least one water potential detector (120) comprises:
i) a compartment with an osmoticum and at least one selective barrier for measuring water potential in the plant stem (21) in which it is installed via direct fluid osmosis, said at least one water potential detector (120) being configured for communicating with said central unit (150) via at least one communication link for transmitting data thereto indicative of the measured water potential;
ii) a thermometer, wherein the water potential detector (120) is configured for transmitting temperature measurements to the central unit (150);
iii) a battery and a communication unit configured for wireless communication with the central unit (150), wherein said communication unit is optionally adapted for radio frequency (RF) based communication; or
iv) nodes for connecting to a communication unit for communicating with said central unit (150),
or any combination thereof.

3. The system of claim 1 or 2, wherein said central unit (150) is further configured for:
i) controlling irrigation of the crop plants according to the evaluated irrigation condition of the crop; or
ii) transmitting data indicative of the evaluated irrigation condition of the crop to an irrigation system for controlling irrigation of the crop according to the evaluated irrigation condition thereof.

4. The system according to any one of claims 1 to 3, wherein said at least one water potential detector (120) comprises multiple water potential detectors each installed in a different plant of the crop at locations that are adapted to optimize measurements in relation to the number of water potential detectors and the size of the crop area and crop type.

5. The system according to any one of claims 1 to 4, wherein said central unit (150) is a computer and communication device having a designated control application operable therethrough for carrying out the data processing using at least one evaluation algorithm for the irrigation condition evaluation and calibration.

6. A method for evaluating irrigation condition in crops using thermal imagery comprising:
a) providing a system for evaluating irrigation condition in crops using thermal imagery according to any one of claims 1 to 5;
b) receiving data (41) from the at least one thermal imagery system (110a) configured and positioned for thermal mapping of the crop area;
c) receiving data (42) from the at least one water potential detector (120) installed in a plant stem (21) in a plant of the crop, indicative of water potential of the plant stem (21);
d) calibrating (43) the thermal data from the at least one thermal imagery system (110a) by using the received data from the water potential detectors (120); and
e) evaluating (44) irrigation condition of the crop based on the calibration data.

7. The method according to claim 6, wherein said evaluation of the irrigation condition of the crop is carried out by also using the water potential data received from the at least one water potential detector (120).

## Patentansprüche

1. System zum Evaluieren eines Bewässerungszustands in Nutzpflanzen unter Verwendung von Wärmebildern, wobei das System umfasst:
a) mindestens ein Wärmebildsystem (110a), das zur thermischen Kartierung eines Bereichs konfiguriert ist;
b) mindestens einen Wasserpotentialdetektor (120), der zum Messen des Wasserpotentials in einem Pflanzenstängel (21), in dem er installiert ist, und Übertragen von Daten, die seine Messungen angeben, konfiguriert ist; und
c) eine Zentraleinheit (150), die zum Empfangen von Wärmebilddaten, die erfasste Nutzpflanzentemperaturkarten angeben, Empfangen von Daten von dem mindestens einen Wasserpotentialdetektor (120) und Verarbeiten der empfangenen Daten zum Evaluieren des Bewässerungszustands der Nutzpflanze konfiguriert ist, **dadurch gekennzeichnet dass** die Zentraleinheit (150) zum Evaluieren des Bewässerungszustands in Nutzpflanzen unter Verwendung der Daten von der mindestens einen Wasserpotentialdetektorreferenz zum Kalibrieren der Daten vom Wärmebildsystem (110a) konfiguriert ist.

2. System nach Anspruch 1, wobei jeder des mindestens einen Wasserpotentialdetektors (120) umfasst:
i) eine Kammer mit einem Osmotikum und mindestens einer selektiven Barriere zum Messen des Wasserpotentials im Pflanzenstängel (21), in dem er installiert ist, mittels direkter Flüssigkeitsosmose, wobei der mindestens eine Wasserpotentialdetektor (120) zum Kommunizieren mit der Zentraleinheit (150) über mindestens eine Kommunikationsverbindung zum Übertragen von Daten zu dieser, die das gemessene Wasserpotential angeben, konfiguriert ist;
ii) ein Thermometer, wobei der Wasserpotentialdetektor (120) zum Übertragen von Temperaturmessungen zu der Zentraleinheit (150) konfiguriert ist;
iii) eine Batterie und eine Kommunikationseinheit, die für eine drahtlose Kommunikation mit der Zentraleinheit (150) konfiguriert ist, wobei die Kommunikationseinheit optional für eine auf Hochfrequenz (HF)-basierte Kommunikation eingerichtet ist; oder
iv) Knoten zur Verbindung mit einer Kommunikationseinheit zum Kommunizieren mit der Zentraleinheit (150),
oder eine beliebige Kombination davon.

3. System nach Anspruch 1 oder 2, wobei die Zentraleinheit (150) ferner konfiguriert ist zum:
i) Steuern der Bewässerung der Nutzpflanzen gemäß dem evaluierten Bewässerungszustand der Nutzpflanze; oder
ii) Übertragen von Daten, die den evaluierten Bewässerungszustand der Nutzpflanze angeben, zu einem Bewässerungssystem zur Steuerung der Bewässerung der Nutzpflanze gemäß dem evaluierte Bewässerungszustand von dieser.

4. System nach einem der Ansprüche 1 bis 3, wobei der mindestens eine Wasserpotentialdetektor (120) mehrere Wasserpotentialdetektoren umfasst, die jeweils in einer anderen Pflanze der Nutzpflanzen an Orten installiert sind, die zum Optimieren von Messungen in Bezug auf die Anzahl von Wasserpotentialdetektoren und die Größe des Nutzpflanzenbereichs und des Nutzpflanzentyps eingerichtet sind.

5. System nach einem der Ansprüche 1 bis 4, wobei die Zentraleinheit (150) eine Computer- und Kommunikationsvorrichtung ist, die eine dadurch betreibbare designierte Steueranwendung zum Ausführen der Datenverarbeitung unter Verwendung mindestens eines Evaluierungsalgorithmus für die Bewässerungszustandsevaluierung und Kalibrierung aufweist.

6. Verfahren zum Evaluieren des Bewässerungszustands in Nutzpflanzen unter Verwendung von Wärmebildern, umfassend:
a) Bereitstellen eines Systems zum Evaluieren des Bewässerungszustands in Nutzpflanzen unter Verwendung von Wärmebildern nach einem der Ansprüche 1 bis 5;
b) Empfangen von Daten (41) von dem mindestens einen Wärmebildsystem (110a), das zur thermischen Kartierung des Nutzpflanzenbereichs konfiguriert und positioniert ist;
c) Empfangen von Daten (42) von dem mindestens einen Wasserpotentialdetektor (120), der in einem Pflanzenstängel (21) in einer Pflanze der Nutzpflanzen installiert ist, die das Wasserpotential des Pflanzenstängels (21) angeben;
d) Kalibrieren (43) der Wärmedaten von dem mindestens einen Wärmebildsystem (110a) unter Verwendung der empfangenen Daten von den Wasserpotentialdetektoren (120); und
e) Evaluieren (44) des Bewässerungszustands der Nutzpflanze basierend auf den Kalibrierungsdaten.

7. Verfahren nach Anspruch 6, wobei die Evaluierung des Bewässerungszustands der Nutzpflanze auch unter Verwendung der Wasserpotentialdaten ausgeführt wird, die von dem mindestens einen Wasserpotentialdetektor (120) empfangen werden.

## Revendications

1. Système pour évaluer une condition d'irrigation dans des cultures à l'aide d'une imagerie thermique, ledit système comprenant :
a) au moins un système d'imagerie thermique (110a) configuré pour réaliser une cartographie thermique d'une zone ;
b) au moins un détecteur de potentiel hydrique (120) configuré pour mesurer un potentiel hydrique dans une tige de plante (21) dans laquelle il est installé et transmettre des données indicatives de ses mesures ; et
c) une unité centrale (150) configurée pour recevoir des données d'imagerie thermique indicatives de cartes de température de culture acquises, recevoir des données provenant de l'au moins un détecteur de potentiel hydrique (120) et pour traiter les données reçues pour évaluer une condition d'irrigation de la culture, **caractérisé en ce que**
l'unité centrale (150) est configurée pour évaluer une condition d'irrigation dans des cultures à l'aide des données provenant de l'au moins une référence de détecteur de potentiel hydrique pour étalonner les données provenant du système d'imagerie thermique (110a).

2. Système selon la revendication 1, dans lequel chacun dudit au moins un détecteur de potentiel hydrique (120) comprend :
i) un compartiment ayant un agent osmotique et au moins une barrière sélective pour mesurer un potentiel hydrique dans la tige de plante (21) dans laquelle il est installé via osmose de fluide directe, ledit au moins un détecteur de potentiel hydrique (120) étant configuré pour communiquer avec ladite unité centrale (150) via au moins une liaison de communication pour transmettre des données à celle-ci indicatives du potentiel hydrique mesuré ;
ii) un thermomètre, dans lequel le détecteur de potentiel hydrique (120) est configuré pour transmettre des mesures de température à l'unité centrale (150) ;
iii) une batterie et une unité de communication configurée pour une communication sans fil avec l'unité centrale (150), dans lequel ladite unité de communication est facultativement adaptée pour une communication par radiofréquence (RF) ; ou
iv) des nœuds pour se connecter à une unité de communication pour communiquer avec ladite unité centrale (150),
ou une combinaison de ceux-ci.

3. Système selon la revendication 1 ou 2, dans lequel ladite unité centrale (150) est en outre configurée pour :
i) réguler l'irrigation des plantes cultivées selon la condition d'irrigation évaluée de la culture ; ou
ii) transmettre des données indicatives de la condition d'irrigation évaluée de la culture à un système d'irrigation pour réguler l'irrigation de la culture selon la condition d'irrigation évaluée de celle-ci.

4. Système selon l'une quelconque des revendications 1 à 3, dans lequel ledit au moins un détecteur de potentiel hydrique (120) comprend de multiples détecteurs de potentiel hydrique installés chacun dans une plante différente de la culture à des emplacements qui sont adaptés pour optimiser des mesures en lien avec le nombre de détecteurs de potentiel hydrique et la taille de la zone de culture et le type de culture.

5. Système selon l'une quelconque des revendications 1 à 4, dans lequel ladite unité centrale (150) est un ordinateur et un dispositif de communication ayant une application de régulation désignée apte à fonctionner par le biais de celui-ci pour effectuer le traitement de données à l'aide d'au moins un algorithme d'évaluation pour l'évaluation et l'étalonnage de la condition d'irrigation.

6. Procédé pour évaluer une condition d'irrigation dans des cultures à l'aide d'une imagerie thermique comprenant :
a) la fourniture d'un système pour évaluer une condition d'irrigation dans des cultures à l'aide d'une imagerie thermique selon l'une quelconque des revendications 1 à 5 ;
b) la réception de données (41) provenant de l'au moins un système d'imagerie thermique (110a) configuré et positionné pour la réalisation d'une cartographie thermique de la zone de culture ;
c) la réception de données (42) provenant de l'au moins un détecteur de potentiel hydrique (120) installé dans une tige de plante (21) dans une plante de la culture, indicatives d'un potentiel hydrique de la tige de plante (21) ;
d) l'étalonnage (43) des données thermiques provenant de l'au moins un système d'imagerie thermique (110a) en utilisant les données reçues provenant des détecteurs de potentiel hydrique (120) ; et
e) l'évaluation (44) d'une condition d'irrigation de la culture sur la base des données d'étalonnage.

7. Procédé selon la revendication 6, dans lequel ladite évaluation de la condition d'irrigation de la culture est effectuée en utilisant également les données de potentiel hydrique reçues en provenance de l'au moins un détecteur de potentiel hydrique (120).
